# EUROPEAN PATENT SPECIFICATION

(11) **EP 3 326 971 B1**
(45) Date of publication and mention of the grant of the patent: **02.12.2020**
(21) Application number: 16827304.3
(22) Date of filing: 20.07.2016
(51) Int. Cl.: A01N 37/40, A01N 37/38, A01N 37/02, A01N 35/04, A01N 31/14, A01N 31/08, A01N 25/34, A01N 25/26, A01N 25/10, A01N 25/08, A01P 7/02, A01P 7/04, A01P 3/00, A01P 1/00, C01B 33/18, A61K 31/05, A61K 31/085

(54) **ANTIMICROBIAL, INSECTICIDAL AND ACARICIDAL SYSTEM**
ANTIMIKROBIELLES, INSEKTIZIDES UND AKARIZIDES SYSTEM
SYSTÈME ANTIMICROBIEN, INSECTICIDE ET ACARICIDE

(30) Priority: 21.07.2015 ES 201531075
(43) Date of publication of application: 30.05.2018
(73) Proprietor: Universitat Politècnica de València, 46022 Valencia (ES)
(72) Inventor: BARAT BAVIERA, José Manuel, 46022 Valencia (ES); MARCOS MARTINEZ, María Dolores, 46022 Valencia (ES); MARTINEZ MAÑEZ, Ramón, 46022 Valencia (ES); PEREZ ESTEVE, Edgar, 46022 Valencia (ES); RUIZ RICO, María, 46022 Valencia (ES); SANCENON GALARZA, Félix, 46022 Valencia (ES)
(74) Representative: Maslanka Kubik, Dorota Irena
(86) International application number: PCT/ES2016/070546
(87) International publication number: WO 2017/013294

(56) References cited:
- WO-A1-2011/124739
- WO-A1-2015/044673
- ES-A1- 2 366 841
- ANEZKA JANATOVA ET AL: "Long-term antifungal activity of volatile essential oil components released from mesoporous silica materials", INDUSTRIAL CROPS AND PRODUCTS., vol. 67, 1 May 2015 (2015-05-01), pages 216-220, XP055443108, NL ISSN: 0926-6690, DOI: 10.1016/j.indcrop.2015.01.019
- DATABASE CA [Online] CHEMICAL ABSTRACTS SERVICE, COLUMBUS, OHIO, US; CHEN, FEI ET AL: "Antioxidant and antibacterial activities of eugenol and carvacrol- grafted chitosan nanoparticles", XP002787385, retrieved from STN Database accession no. 152:19255 & CHEN, FEI ET AL: "Antioxidant and antibacterial activities of eugenol and carvacrol- grafted chitosan nanoparticles", BIOTECHNOLOGY AND BIOENGINEERING , 104(1), 30-39 CODEN: BIBIAU; ISSN: 0006-3592, 2009, DOI: 10.1002/BIT.22363 10.1002/BIT.22363
- DATABASE CA [Online] CHEMICAL ABSTRACTS SERVICE, COLUMBUS, OHIO, US; MARIN, LUMINITA ET AL: "Antifungal vanillin-imino-chitosan biodynameric films", XP002787386, retrieved from STN Database accession no. 159:203585 & MARIN, LUMINITA ET AL: "Antifungal vanillin-imino-chitosan biodynameric films", JOURNAL OF MATERIALS CHEMISTRY B: MATERIALS FOR BIOLOGY AND MEDICINE , 1(27), 3353-3358 CODEN: JMCBDV; ISSN: 2050-7518, 2013, DOI: 10.1039/C3TB20558D 10.1039/C3TB20558D
- DATABASE CA [Online] CHEMICAL ABSTRACTS SERVICE, COLUMBUS, OHIO, US; BEDEL, SOPHIE ET AL: "Antibacterial poly(ethylene terephthalate) surfaces obtained from thymyl methacrylate polymerization", XP002787387, retrieved from STN Database accession no. 163:161466 & Sophie Bedel ET AL: "Antibacterial poly(ethylene terephthalate) surfaces obtained from thymyl methacrylate polymerization", JOURNAL OF POLYMER SCIENCE, PART A: POLYMER CHEMISTRY, vol. 53, no. 17, 1 September 2015 (2015-09-01), pages 1975-1985, XP055533787, US ISSN: 0887-624X, DOI: 10.1002/pola.27647
- DATABASE CA [Online] CHEMICAL ABSTRACTS SERVICE, COLUMBUS, OHIO, US; SATO, NAOYA ET AL: "Photosensitive, hydrophilicity-changing silane coupling agents and their use for manufacture of materials having hydroxyalkyl groups on surfaces", XP002787388, retrieved from STN Database accession no. 158:56327 & JP 2012 246225 A (OKAMOTO CHEMICAL INDUSTRY CO., LTD., JAPAN) 13 December 2012 (2012-12-13)
- DATABASE CA [Online] CHEMICAL ABSTRACTS SERVICE, COLUMBUS, OHIO, US; SCHROEDER, MARC ET AL: "Enzymatic coating of lignocellulosic surfaces with polyphenols", XP002787389, retrieved from STN Database accession no. 148:264159 & SCHROEDER, MARC ET AL: "Enzymatic coating of lignocellulosic surfaces with polyphenols", BIOTECHNOLOGY JOURNAL , 2(3), 334-341 CODEN: BJIOAM; ISSN: 1860-6768, 2007, DOI: 10.1002/BIOT.200600209 10.1002/BIOT.200600209
- HASHEMIKIA SAMANEH ET AL.: 'Optimization of tetracycline hydrochloride adsorption on amino modified SBA-15 using response surface methodology.' ANALYTICAL SCIENCES THE JAPAN SOCIETY FOR ANALYTICAL CHEMISTRY vol. 443, ISSN 0021-9797 pages 105 - 114, XP029188723
- ZAMANI F ET AL.: 'Complexes of N,N-bis (salicylidene)4,5-dimethyl-1,2- phenylenediamine immobilized on porous nanomaterials: Synthesis, characterization and study of their antimicrobial activity.' MICROPOROUS AND MESOPOROUS MATERIALS vol. 212, NEW YORK, US, ISSN 1387-1811 pages 18 - 27, XP029219853
- WANG ET AL.: 'Incorporation of the antibacterial agent, miconazole nitrate into a cellulosic fabric grafted with beta-cyclodextrin.' CARBOHYDRATE POLYMERS vol. 72, no. 4, 10 June 2008, BARKING, GB, ISSN 0144-8617 pages 695 - 700, XP022512211

## Description

### FIELD OF THE INVENTION

The present invention relates to the field of antimicrobial, insecticidal and acaricidal compounds, and more specifically to antimicrobial, insecticidal and acaricidal systems for the administration of said compounds.

### BACKGROUND OF THE INVENTION

Plant metabolites such as the active compounds of essential oils and the organic acids of plant extracts exhibit proven acaricidal, antimicrobial and insecticidal activity against different pests ("Antimicrobial gallic acid from Caesalpinia mimosoides Lamk." Food Chemistry, 100(3), 1044-1048, 2007; "Biological effects of essential oils-a review." Food and Chemical Toxicology, 46(2), 446-475, 2008; "Essential oils in food preservation: mode of action, synergies, and interactions with food matrix components." Frontiers in Microbiology, 3, 2012). The use of these extracts and the active compounds thereof as phytosanitary agents in crop farming, post-harvest treatments and in the food industry as antimicrobial agents in foods, coatings and containers has been widely studied in recent years ("Essential oils: their antibacterial properties and potential applications in foods-a review." International journal of food microbiology, 94(3), 223-253, 2004; "Commercial opportunities for pesticides based on plant essential oils in agriculture, industry and consumer products." Phytochemistry Reviews, 10(2), 197-204, 2011).

The main limitations of the use of compounds of this type are their solubility and volatility, as well as the modification of the organoleptic characteristics of foods after their incorporation, mainly the strong smell which prevents their use at effective concentrations for food preservation.

Encapsulation of compounds of this type in mesoporous silicon oxide particles as an alternative dosage form has recently started to be the object of study.

In the literature, mesoporous silicon oxide particles, or MSPs, are considered biocompatible materials, as inferred, for example, from the study conducted by Wehling et al. ("A critical study: Assessment of the effect of silica particles from 15 to 500 nm on bacterial viability." Environmental Pollution, 176, 292-299, 2013), which investigated the effect of silicon oxide particles with a size comprised between 15 and 500 nm on bacterial viability. The results established that the particles did not exhibit inhibitory properties regardless of their particle size. Other research which has established the antibacterial effect of loaded particles also showed that the unloaded supports did not have any inhibitory activity on the studied microorganisms.

Loading antimicrobial compounds into the pores of mesoporous silicon oxide particles has been widely studied. For example, in the article by Izquierdo-Barba et al. ("Incorporation of antimicrobial compounds in mesoporous silica film monolith." Biomaterials, 30(29), 5729-5736, 2009), the antimicrobial peptide LL-37, as well as chlorhexidine, were encapsulated in mesoporous silicon oxide and the controlled release thereof was obtained by means of anchoring -SH groups on the surface of silicon oxide. Both loaded particles showed bactericidal activity against Gram-positive and Gram-negative bacteria, with the chlorhexidine-loaded particles being more toxic according to the hemolysis, lactate dehydrogenase release and cell viability tests.

Additionally, nitroimidazole PA-824, which has a high antituberculous activity, was encapsulated in mesoporous silicon oxide particles, improving its solubility and therefore its bioavailability. However, the antibacterial activity of this encapsulated compound was not greater than the effect produced by the free compound. The absence of improvement in the activity may have been due to difficulties during PA-824 release from the mesoporous particles as a result of molecules which may interfere with the cellular environment ("Encapsulation of anti-tuberculosis drugs within mesoporous silica and intracellular antibacterial activities." Nanomaterials, 4, 813-826, 2014).

An example of the encapsulation of natural antimicrobial compounds from plant extracts is allyl isothiocyanate. This natural antibacterial compound could be used in food, but presents problems relating to its volatility, spicy flavor and low water solubility. The encapsulation of this compound in mesoporous silicon oxide particles was proposed and controlled release of allyl isothiocyanate, which was modulated according to pore size distribution, was obtained. The antibacterial properties of said compound were maintained following the adsorption and desorption processes ("Controlled release of allyl isothiocyanate for bacteria growth management." Food Control, 23(2), 478-484, 2012).

It is also known encapsulation of volatile essential oil components into mesoporous silica particles for their controlled release to provide a prolonged antifungal effect from ANEZKA JANATOVA ET AL, "Long-term antifungal activity of volatile essential oil components released from mesoporous silica materials", INDUSTRIAL CROPS AND PRODUCTS., NL, (20150501), vol. 67, doi:10.1016/j.indcrop.2015.01.019, ISSN 0926-6690, pages 216 - 220. In this case, antimicrobial compounds are entrapped into the pores of mesoporous material. In order to exert their biocidal activity, the bioactive compounds should be released.

It is also known encapsulation of antimicrobial agents, such as cinnamaldehyde, into mesoporous core material for their protection from WO2015044673. According to its disclosure, the antimicrobial compounds are entrapped into the pores of mesoporous material. In order to exert their biocidal activity, the bioactive compounds should be released.

However, although the encapsulation of active compounds inside mesoporous particles provides advantages in terms of controlled and sustained release of the compound, for example, these techniques are not completely satisfactory. For example, one drawback of a technique of this type is that once the active compounds are released from the mesoporous particles, they are free in the environment and therefore have the same drawbacks as said compounds administered individually (for example, in terms of solubility, volatility with reduced efficacy against pests due to the low persistence, unpleasant flavor, etc.). Additionally, as the released compounds are dispersed in the environment, a substantial concentration of said compounds in a specific region of interest is not obtained. Additionally, it is not always possible to introduce the mentioned compounds into the pores due to polarity ratios and pore size-to-compound size ratio. On the other hand, the use of porous substances with pores having a large diameter complicates the closing thereof and therefore the effective encapsulation of the added compound.

When administered to a patient (which can be a human being or another animal species) for the treatment of a disease, for example, the active compounds can be absorbed in the body of the patient once they have been released. This prevents or reduces the action of those compounds in subsequent regions of the gastrointestinal tract, such as in the large intestine or the cecum, for example.

On the other hand, there are also known chitosan nanoparticles functionalized with eugenol and carvacrol with antioxidant and antibacterial activities as disclosed in CHEN, FEI ET AL, "Antioxidant and antibacterial activities of eugenol and carvacrol- grafted chitosan nanoparticles", CA, CHEMICAL ABSTRACTS SERVICE, COLUMBUS, OHIO, US, Database accession no. 152:19255. The antimicrobial compounds are grafted to the chitosan particles by reaction between the amino groups naturally present in chitosan and the aldehyde of previous derivatized carvacrol and eugenol. However, the imine bond formed between the aldehyde of the bioactive compounds and the amino moiety of chitosan is easily hydrolysable at slight acidic pH, with the subsequent release of the grafted compound.

There are also known chitosan biodynameric films functionalized with vanillin with antifungal properties as disclosed in MARIN, LUMINITA ET AL, "Antifungal vanillin-imino-chitosan biodynameric films", CA, CHEMICAL ABSTRACTS SERVICE, COLUMBUS, OHIO, US, Database accession no. 159:203585. Vanillin is grafted to the chitosan particles by reaction between the amino groups naturally present in chitosan and the aldehyde of moiety of vanillin. The imine bond formed between the aldehyde moiety of vanillin and the amino moiety of chitosan is easily hydrolysable at slight acidic pH, with the subsequent release of the grafted compound.

There are also known PET films functionalized with thymol by using a methacrylic monomer of thymol as disclosed in BEDEL, SOPHIE ET AL, "Antibacterial poly(ethylene terephthalate) surfaces obtained from thymyl methacrylate polymerization", CA, CHEMICAL ABSTRACTS SERVICE, COLUMBUS, OHIO, US, Database accession no. 163:161466. PET film is firstly treated with NH3 plasma to incorporate primary amino groups. Then, the thymol methacrylate is polymerized onto the surface of PET film by immobilization of an atom transfer radical initiator. In this case, attachment of thymol by means of the hydroxyl group would imply the loss of its antimicrobial features due to the key role of this moiety on the interaction with the microbial envelope to generate irreversible cell damage.

There are also known compounds containing photosensitive and hydrophobic moieties functionalized with trialkoxysilanes to generate materials with hydroxyalkyl groups on the surface after irradiation with 365 nm wavelength light as disclosed in SATO, NAOYA ET AL, "Photosensitive, hydrophilicity-changing silane coupling agents and their use for manufacture of materials having hydroxyalkyl groups on surfaces", CA, CHEMICAL ABSTRACTS SERVICE, COLUMBUS, OHIO, US, Database accession no. 158:56327. To attach certain molecular entities (ca. essential oils) onto the surface it is necessary the irradiation step in order to generate hydroxyl moieties in which the molecules could be grafted. This mandatory irradiation step limits the applications of the described materials.

There are also known lignocellulosic surfaces functionalized with polyphenols by enzymatic oxidative coupling as disclosed in SCHROEDER, MARC ET AL, "Enzymatic coating of lignocellulosic surfaces with polyphenols", CA, CHEMICAL ABSTRACTS SERVICE, COLUMBUS, OHIO, US, Database accession no. 148:264159. The oxidative coupling used to immobilize polyphenol is induced by radicals generated by certain oxidase-reductase enzymes. These radical processes are difficult to control and, therefore, homogeneity in the degree of functionalization cannot be ensured.

There are also known silica nanoparticles functionalized with bioactive agents (amino acid, oligopeptide, antibody, protein, enzyme or polymer) with low solubility as disclosed in WO2011124739. The materials consist of two components: (i) the silica nanoparticles with anionic terminal moieties and (ii) the bioactive agent covalently grafted to the anionic groups on the surface of the inorganic support. The enzymatic or chemical hydrolysis of this covalent bond (ester, amide, carbamate or carbonate) induces the controlled release of the insoluble bioactive agent for biomedical applications. This is an example of smart delivery system in which the bioactive compounds are grafted but their biological effect takes place once the bioactive agent is released.

Therefore, there is still a need in the art for an alternative antimicrobial system which allows obtaining a substantial concentration of an active compound in a region of interest. Furthermore, it would be desirable for the antimicrobial system to allow masking and reducing, at least partially, certain undesired properties of antimicrobial compounds (for example, unpleasant flavor and smell, etc.) in the case of food applications, for example.

### SUMMARY OF THE INVENTION

To solve the problems of the prior art and to assure the maintenance of the antimicrobial, insecticidal and acaricidal properties, the present invention proposes anchoring specific antimicrobial, insecticidal and acaricidal compounds (indicated hereinafter as active compounds) on the surface of support particles.

In principle, said anchoring or functionalization can greatly reduce the antimicrobial, insecticidal and acaricidal activity of the active compounds since the most reactive functional groups, which are those through which the active compounds are usually anchored to the solid, are groups which confer the antimicrobial, insecticidal and acaricidal character to said compounds. However, the inventors have now surprisingly discovered that in certain specific groups of active compounds of natural origin, such a decrease in activity does not occur when they are anchored on a solid support, rather said activity has been observed to remain the same or to increase. Even in the case of the same antimicrobial, insecticidal or acaricidal activity being maintained, the anchoring of the compounds on a solid support will provide additional advantages, such as the masking of unpleasant smells and flavors, for example.

The present invention therefore discloses an antimicrobial, insecticidal and acaricidal system comprising support surface-functionalized by derivatization with trialkoxysilane groups, with at least one specific antimicrobial, insecticidal or acaricidal compound of natural origin covalently attached to said support. According to the present invention, the active compounds are not loaded into the pores of the support particles or support surface like in the case of the prior art, rather they are functionalized on the surface of said support. The compounds therefore remain anchored to the support and are not released into the medium, which provides a series of advantages with respect to the antimicrobial, insecticidal and acaricidal systems of the prior art.

Therefore, given that the active compounds are not released into the environment, undesirable properties of the compounds, such as the unpleasant smell and flavor, are masked, their volatility is reduced, etc. Furthermore, given that these compounds are not dispersed in the medium to which they are supplied, their concentration increases at the site of administration of the particles, for example, and therefore the amount of said compounds that must be provided to obtain a given effect is reduced.

On the other hand, as the compounds are permanently fixed to the mesoporous particles (supports as described in the present invention), their absorption in the intestinal tract when administered to a patient is prevented. This favors the action of the active compounds in subsequent regions of the gastrointestinal tract, such as the large intestine or the cecum.

According to a preferred embodiment of the present invention, the support used in the antimicrobial system are particles selected from the group consisting of silica-based inorganic materials, preferably mesoporous silicon oxide particles with a particle size comprised between 0.01 and 7 µm and a pore size comprised between 1 and 20 nm.

According to another embodiment of the invention, the support particles are amorphous silicon oxide particles with a particle size comprised between 0.01 and 30 µm.

According to another embodiment of the invention, the support are particles selected from the group consisting of cellulose particles, preferably microcrystalline cellulose particles with a particle size comprised between 1 and 10 µm. The present invention also discloses the use of cellulose-rich supports, such as wood, cotton or paper, for example.

Additionally, according to the present invention, the active compound used in the antimicrobial, insecticidal or acaricidal system is selected from the group consisting of specific active compounds present in plant essential oils viz. carvacrol, cinnamaldehyde, perillaldehyde, eugenol, thymol and vanillin and specific organic acids present in the aqueous phase of plant extracts viz. gallic acid and ferulic acid.

The present invention also discloses that at least one active compound of natural origin can be applied to the support by means of spraying.

The present invention also discloses the use of the system according to the present invention as antimicrobial filters and antimicrobial fabrics.

### BRIEF DESCRIPTION OF THE DRAWINGS

The present invention will be better understood in reference to the following drawings.
Figure 1 shows two bar graphs illustrating the inhibition of *L. innocua* obtained with free vanillin (control) and with vanillin functionalized on support particles, as a function of concentration.
Figure 2 shows two bar graphs illustrating the inhibition of *L. innocua* obtained with free carvacrol (control) and with carvacrol functionalized on support particles, as a function of concentration.
Figure 3 shows TEM microphotographs of ultrathin sections of *L. innocua* in the presence of MCM-41 particles functionalized with active compounds.
Figure 4 shows the inhibitory activity of free eugenol and eugenol anchored to amorphous silicon oxide (dark gray bars) and MCM-41 microparticles (light gray bars) on *Listeria innocua* (A, B) and *Escherichia coli* (C, D).
Figure 5 shows the inhibitory activity of free thymol and thymol anchored to amorphous silicon oxide (dark gray bars) and MCM-41 microparticles (light gray bars) on *Listeria innocua* (A, B) and *Escherichia coli* (C, D).
Figure 6 shows the inhibitory activity of free eugenol and eugenol anchored to MCM-41 microparticles on *Aspergillus flavus* (A, B), *Aspergillus niger* (C, D) and *Penicillium expansum* (E, F).
Figure 7 shows the inhibitory activity of free eugenol and eugenol anchored to MCM-41 microparticles on *Zygosaccharomyces bailii* (A, B) and *Zygosaccharomyces rouxii* (C, D).
Figure 8 shows the progression of the movement of *Tyrophagus putrescentiae* in the presence of free carvacrol, eugenol and thymol (A) and carvacrol, eugenol and thymol anchored to amorphous silicon oxide particles (B).
Figure 9 shows the inhibitory activity of free thymol (A) and free vanillin (B) (light gray bars) and thymol and vanillin anchored to microcrystalline cellulose (dark gray bars) in *Listeria innocua.*
Figure 10 shows images of the growth or absence of growth of *Aspergillus niger* in control cotton fabric (a), fabric functionalized with thymol (b) and fabric functionalized with vanillin (c).

### DETAILED DESCRIPTION OF PREFERRED EMBODIMENTS

As mentioned above, the present invention relates to an antimicrobial, insecticidal and acaricidal system made up of support surface-functionalized by derivatization with trialkoxysilane groups, with at least one antimicrobial, insecticidal and acaricidal compound of natural origin covalently attached to said support.

According to an embodiment of the present invention, the antimicrobial, insecticidal and acaricidal system comprises support particles functionalized by derivatization with trialkoxysilane groups, with an antimicrobial, insecticidal or acaricidal compound of natural origin as defined above and in claim 1 covalently attached on the surface thereof.

According to another embodiment of the present invention, the antimicrobial, insecticidal and acaricidal system comprises support particles functionalized by derivatization with trialkoxysilane groups, with two or more different antimicrobial compounds of natural origin as defined above and in claim 1 covalently attached on the surface thereof, such that the system facilitates the simultaneous action of both compounds.

According to a preferred embodiment, the support are particles that are selected from the group consisting of silica-based inorganic materials, preferably mesoporous silicon oxide particles with a particle size comprised between 0.01 and 7 µm, preferably between 0.1 and 1.5 µm; and a pore size comprised between 1 and 20 nm, preferably between 2 and 8 nm. More preferably, the mesoporous silicon oxide particles are preferably selected from MCM-41, SBA-15 and UVM-7.

According to another preferred embodiment, the support particles are amorphous silicon oxide particles with a particle size comprised between 0.01 and 30 µm, preferably between 3 and 4 µm.

According to another embodiment of the invention, the support are particles selected from the group consisting of cellulose particles, preferably microcrystalline cellulose particles with a particle size comprised between 1 and 10 µm. Said particles have been used up until now in the pharmaceutical and food industry as an excipient and additive (E-460) with a stabilizing and thickening function, but their use as support particles is unknown.

According to another embodiment of the invention, the functionalized support is selected from the group of cellulose-rich supports, such as wood, cotton and paper, for example.

In turn, the antimicrobial, insecticidal and acaricidal compound of natural origin functionalized on the support is as defined above and in claim 1. More preferably, the antimicrobial compound functionalized on the support is selected from the group consisting of carvacrol, eugenol, thymol and vanillin.

The present invention also relates to the use of the system according to the present invention as antimicrobial filters and antimicrobial fabrics. At least one active compound of natural origin can also be applied to the desired support by means of spraying.

### IMMOBILIZATION PROTOCOL

The immobilization of natural bioactive compounds must be suitably studied and performed, since there is a relationship between the reactivity of the functional groups of the bioactive molecules and their biological activity potential. For that reason, not only is it necessary to anchor the mentioned molecules, but sometimes approaches which allow keeping the active group of the bioactive compound free must be employed.

The inorganic supports object of this patent have a large specific surface area with reactive groups on the surface thereof which allows the efficient covalent anchoring of organic molecules.

The chemical anchoring of various plant extract components as defined above and in claim 1 on inorganic supports will allow maintaining the biological activity of the compounds by minimizing the adverse effects that may result from applying free compounds. Potential advantages include greater persistence since volatility of the bioactive compounds is reduced after covalent anchoring; reduction in the minimum concentration required for the bioactive compound to produce an inhibitory effect since anchoring the molecules on a surface causes the activity to increase due to a local concentration effect; as well as an increase in the chemical and thermal stability of the bioactive compounds.

The simplest way to anchor these compounds on inorganic supports consists of derivatizing them with trialkoxysilane groups using the main functional groups. Secondly, the derivatized compounds will be anchored on the inorganic surface of the support by means of the formation of covalent bonds.

In the case of anchoring aromatic compounds, formylation (Vilsmeier and Reimer-Tiemann) reactions will be used and the introduced aldehyde group will serve as an anchoring point of the compound with propylamines for the formation of alkoxysilane derivatives that bind to the support material. The aromatic compounds can also be directly anchored to the support material after reaction with iodopropyl groups by means of Friedel-Crafts reactions.

For organic acids having a carboxylic acid as the main functional group, said carboxylic acid will be immobilized on a material prefunctionalized with propylamines by means of an amidation reaction using DCC or EDC as coupling agents.

Illustrative but non-limiting examples are provided below to help understand the present invention.

### EXAMPLES

### Example 1:

First the functionalization of vanillin on MCM-41 mesoporous microparticles, as well as the antimicrobial activity thereof, were studied.

To that end, *in vitro* assays were carried out to determine the bacterial viability of *Listeria innocua* compared to mesoporous particles functionalized with vanillin as well as free vanillin. The survival of the microorganism in the absence of particles or active compound (positive control) was established as 100% of the population to calculate survival in the treated samples.

To that end, different suspensions of particles or free vanillin in culture broth (tryptone soy broth) were prepared, and the microbial inoculum was added thereto to obtain an initial population of about 10⁶ CFU/ml. Said suspensions were incubated under orbital stirring (150 rpm) at 37°C for 2 h. After said period, the serial dilutions were seeded in selective medium and incubated for 48 h, with subsequent counting of the colony forming units (CFUs) and calculation of the percentage of inhibition.

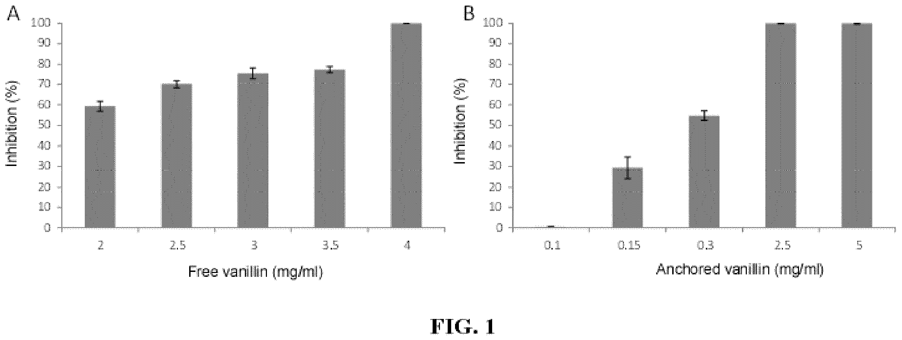

The attached Figure 1 shows the percentages of inhibition on *Listeria innocua* cultures obtained with different concentrations of free vanillin (graph on the left) and vanillin functionalized on MCM-41 particles (graph on the right). As can be seen in Figure 1, the antimicrobial activity of vanillin improves after anchoring to the support compared to the results of free vanillin. The free vanillin produces complete inhibition of the microorganism at a concentration of 4 mg/ml. However, the functionalization of vanillin on MCM-41 particles causes a reduction in the inhibitory concentration, complete inhibition occurring at a concentration of only 2.5 mg/ml of anchored vanillin.

In fact, the concentration of vanillin in a small space as a result of the functionalization on MCM-41 particles greatly increases the antimicrobial capacity of the systems in the presence of free vanillin.

Therefore, the functionalization of vanillin on MCM-41 particles reduces the active compound concentration required for achieving 100% inhibition of an *L. innocua* culture to almost half. Furthermore, said functionalized support particles can be readily suspended in aqueous media, thereby improving vanillin solubility, and hardly have the characteristic smell of the active compound.

A test to determine microorganism inhibition by non-functionalized particles was carried out in the experiments, and no inhibitory effect whatsoever was observed.

### Example 2:

A study similar to that described in Example 1 was performed to determine the antimicrobial activity of carvacrol functionalized on MCM-41 microparticles compared to free carvacrol.

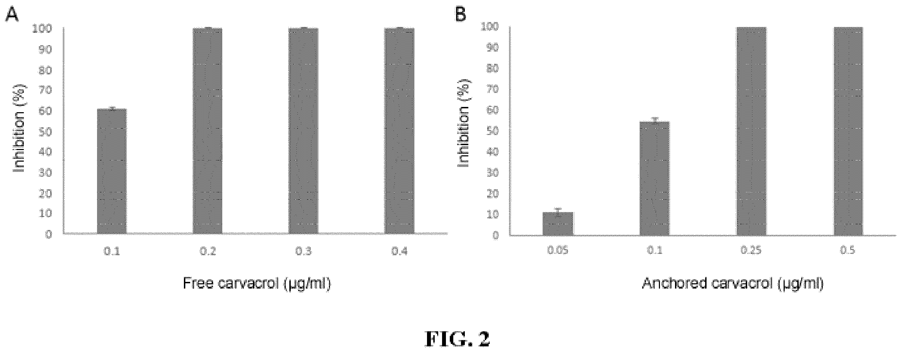

The attached Figure 2 shows the percentages of inhibition on *Listeria innocua* cultures obtained with different concentrations of free carvacrol (graph on the left) and carvacrol functionalized on MCM-41 particles (graph on the right) .

As seen in Figure 2, the antimicrobial activity of carvacrol is maintained after anchoring on silicon oxide microparticles the same way as in the preceding example.

Results similar to those described above in Examples 1 and 2 have also been obtained using support particles of different sizes (data not shown).

### Example 3:

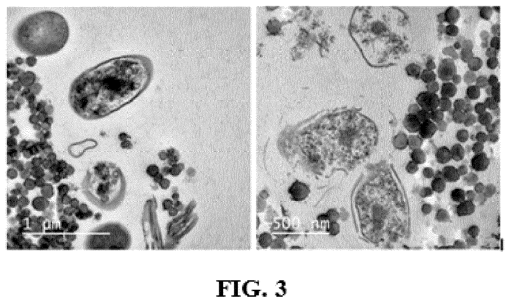

A study was performed by means of transmission electron microscopy (TEM) on the possible mechanism of action of the antimicrobial compounds functionalized on support particles. Figure 3 shows the morphological changes of *L. innocua* cells treated with functionalized MCM-41 particles.

It can be observed that the treated cells exhibit serious morphological damage with a ruptured membrane and cell wall, as well as a loss of intracellular components. These results confirm that, like their free form, the main target of the active compounds functionalized on support particles is the outer cell envelope.

### Example 4 (Comparative Example):

The anchoring of caprylic acid on mesoporous MCM-41 particles was studied. Caprylic acid is a medium-chain fatty acid with proven antimicrobial activity against a wide range of microorganisms, but its use is limited by the strong rancid smell. The anchoring of caprylic acid on MCM-41 microparticles was therefore considered as an alternative for the purpose of maintaining the antimicrobial activity of the fatty acid, preventing the sensory-wise rejection associated with the administration of caprylic acid in free form. The anchoring of the fatty acid was carried out by means of the reaction between the terminal carboxylic group thereof and the amino group previously immobilized on the MCM-41 support, obtaining a support with hydrocarbon chains with the terminal methyl group. However, this anchoring mechanism greatly reduced the antimicrobial properties thereof, where large amounts of functionalized support are required to partially inhibit a bacterial culture (results not shown).

It is therefore demonstrated that not all antimicrobial compounds of natural origin exhibit an enhanced antimicrobial activity by means of functionalization on mesoporous particles disclosed in the present invention, and that the functionalization method used for the anchoring thereof is a decisive factor for the maintenance of their antimicrobial activity.

In fact, it is known that the antimicrobial activity of said compounds is due to certain functional groups in their molecular structure, and therefore the modification of said structure may modify the antimicrobial properties thereof. Particularly, the antimicrobial activity of the active compounds of essential oils is due to the presence of free hydroxyl groups and the blocking of these functional groups causes the loss of the inhibitory effect ("Antimicrobial activity of carvacrol related to its chemical structure." Letters in Applied Microbiology, 43(2), 149-154, 2006).

### Example 5 (Comparative Example):

Nevertheless, the anchoring of caprylic acid on mesoporous MCM-41 particles according to the present invention through a functional group other than the one used in Comparative Example 4 will indeed provide an increase in, or at least the maintenance of, the activity of this antimicrobial compound.

The functionalization route used for preparing this material consisted of a first step in which the inorganic support was functionalized with amino groups. In a second step, the amino groups were reacted with succinic anhydride, producing materials with a high density of carboxylic acids on the outer surface thereof, and 8-hydroxycaprylic acid was finally anchored by means of an esterification reaction.

The material obtained by means of this method had hydrocarbon chains with the terminal carboxylic group on the surface thereof. The tests performed showed that said support maintained the antimicrobial activity of caprylic acid in the same range as the free compound.

### Example 6:

First the functionalization of eugenol on support particles of mesoporous silicon oxide and amorphous silicon oxide and the antimicrobial activity thereof were studied.

To that end, *in vitro* assays were carried out to determine the bacterial viability of *Listeria innocua* and *Escherichia coli* compared to mesoporous particles functionalized with eugenol as well as free eugenol. The survival of the microorganism in the absence of particles or active compound (positive control) was established as 100% of the population to calculate survival in the treated samples.

To that end, different suspensions of free and anchored compound in culture broth (tryptone soy broth) were prepared, and the microbial inoculum was added thereto to obtain an initial population of about 10⁶ CFU/ml. Said suspensions were incubated under stirring at 37°C for 24 h. After said period, the serial dilutions were seeded in selective medium and incubated for 24-48 h, with subsequent counting of the colony forming units (CFUs) and calculation of the percentage of inhibition.

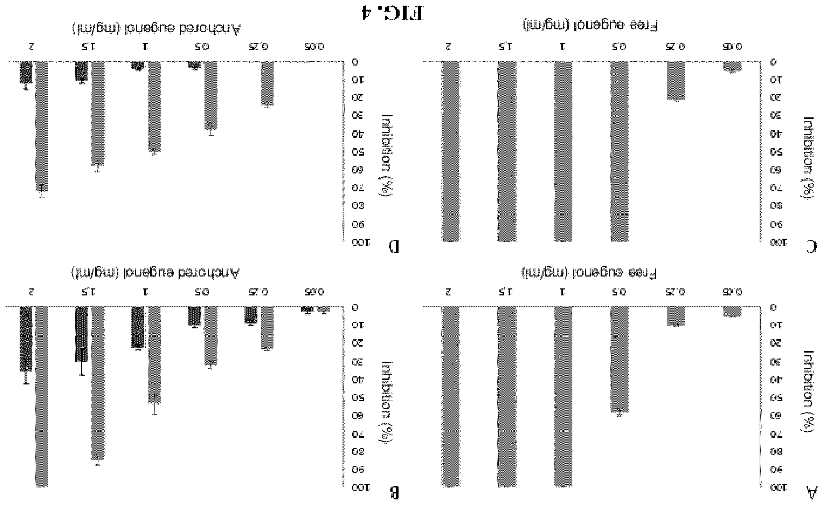

The attached Figure 4 shows the percentages of inhibition on *Listeria innocua* cultures obtained with different concentrations of free eugenol (graph A) and eugenol functionalized (graph B) on amorphous silicon oxide (dark gray bars) and MCM-41 particles (light gray bars). Figure 4 also shows the percentages of inhibition on *Escherichia coli* cultures obtained with different concentrations of free eugenol (graph C) and eugenol functionalized (graph D) on amorphous silicon oxide (dark gray bars) and MCM-41 particles (light gray bars).

As can be seen in Figure 4, the antimicrobial activity of eugenol anchored on mesoporous silicon oxide is maintained in a range similar to that of the free compound in the case of *L*. *innocua,* and the antimicrobial activity is slightly reduced in the case of the bacterium *E. coli.* On the other hand, the active compounds anchored on amorphous silicon oxide maintain the antimicrobial activity but to a lesser extent than the free compounds or compounds immobilized on the microparticulate material MCM-41.

### Example 7:

A study similar to that described in Example 6 was performed to determine the antimicrobial activity of thymol functionalized on MCM-41 microparticles and amorphous silicon oxide particles compared to free thymol.

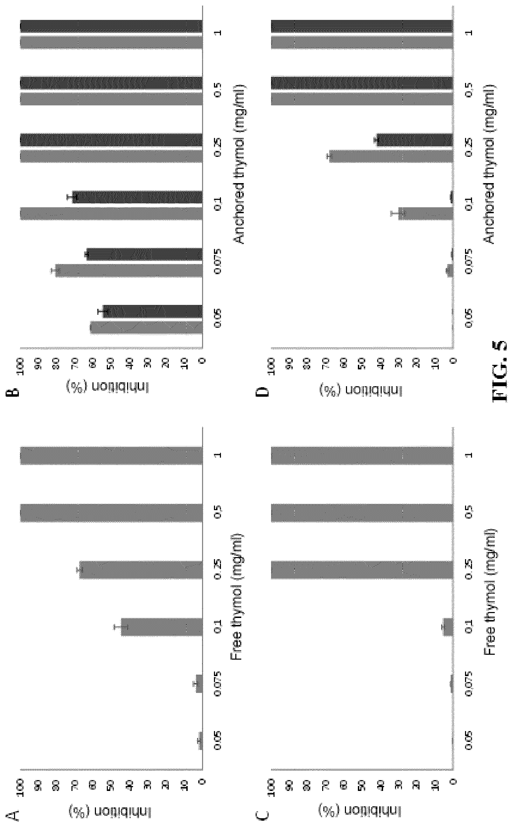

The attached Figure 5 shows the percentages of inhibition on *Listeria innocua* cultures obtained with different concentrations of free thymol (graph A) and thymol functionalized (graph B) on amorphous silicon oxide (dark gray bars) and MCM-41 particles (light gray bars). Figure 5 also shows the percentages of inhibition on *Escherichia coli* cultures obtained with different concentrations of free thymol (graph C) and thymol functionalized (graph D) on amorphous silicon oxide (dark gray bars) and MCM-41 particles (light gray bars).

As seen in Figures 5A and 5B, for *L. innocua* the antimicrobial activity of thymol anchored to both supports is clearly improved compared to the free compound. Thymol concentration on the surface of small particles greatly increases the antimicrobial capacity of the system compared to the compound in its free form.

### Example 8:

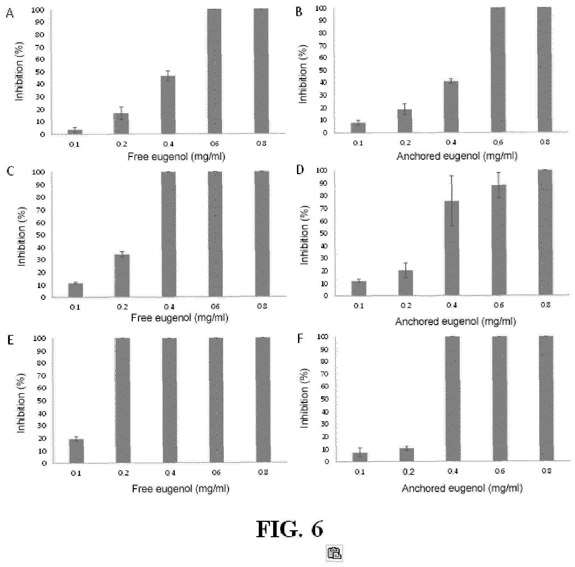

Similarly, an *in vitro* study to determine the anti-mold and anti-yeast activity of eugenol anchored on MCM-41 was performed. Figure 6 shows six bar graphs showing the percentage of inhibition of A. *flavus* (A, B), *A. niger* (C, D) and *P. expansum* (C, D) obtained with free eugenol (control) and with eugenol anchored on MCM-41 support particles, as a function of concentration.

As seen in the drawing, the antimicrobial activity of free eugenol and the eugenol anchored on molds is very similar, so eugenol anchored on the solid support does not reduce the fungicidal activity of the active compound.

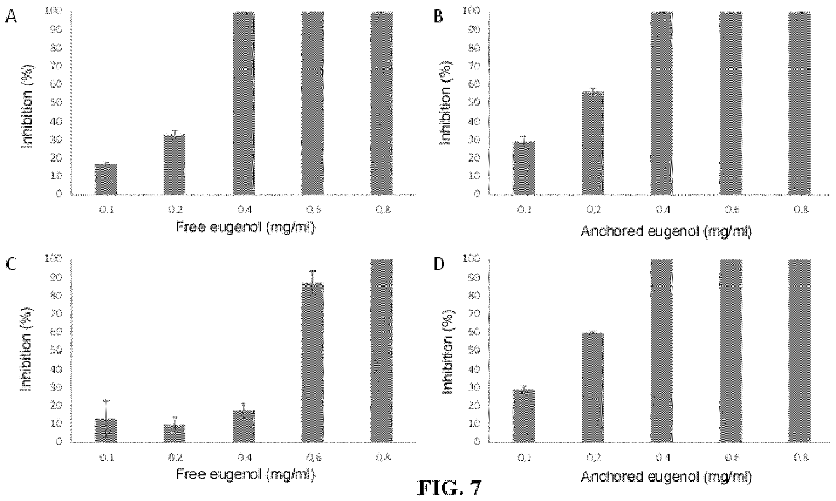

Likewise, the results of inhibition on yeasts *Z*. *bailii* and *Z. rouxii* (Figure 7) show that the anchored compound maintains and even improves the inhibitory activity compared to free eugenol.

### Example 9:

In addition to antimicrobial activity, a study of the acaricidal activity of immobilized active compounds of essential oils has been performed. To carry out these tests, the movements of mites were recorded by means of capturing images in order to establish the displacements and paths of the individuals, and the viability thereof was determined with this data.

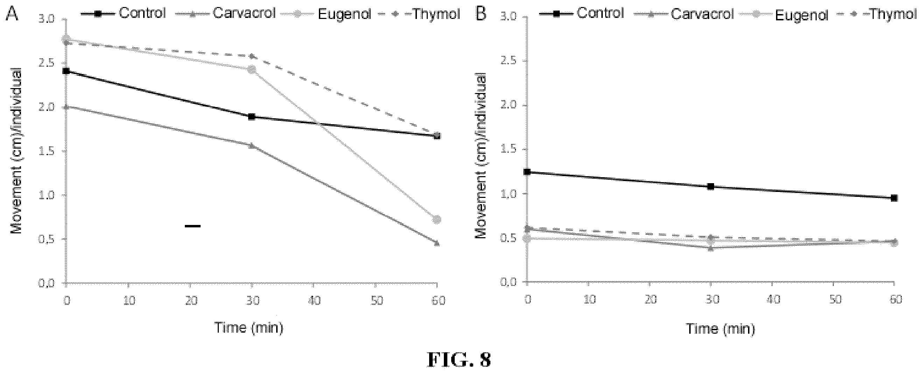

Figure 8 shows a line graph showing the movement of mites in the absence of treatment or with non-functionalized particles (control) and in the presence of the active compounds, i.e., free carvacrol, eugenol and thymol, and carvacrol, eugenol and thymol anchored on amorphous silicon oxide, as a function of the time of treatment.

The results show that the acaricidal activity is maintained after anchoring of the active compound of essential oil as seen in Figure 8, which is certainly promising since the acaricidal activity of compounds of this type has been described up until now after volatilization but not after immobilization on a support.

### Example 10:

The use of microcrystalline cellulose particles as a solid support has been studied.

The anchoring of the active compounds on cellulose was performed following the same protocol for anchoring on silicon oxide supports, only taking into account the solvent suitable for the suspension of cellulose (dimethylsulfoxide).

The results obtained have shown that immobilization takes place correctly and that the active compounds maintain antimicrobial activity.

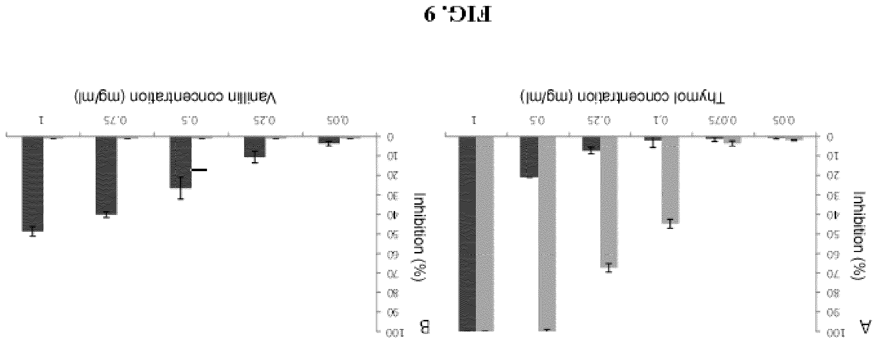

Figure 9 shows the percentage of inhibition of *L. innocua* after treatment with free thymol and vanillin and with thymol and vanillin anchored on microcrystalline cellulose.

As seen in the drawing, microcrystalline cellulose particles functionalized with thymol and vanillin maintain and even improve, like in the case of anchored vanillin, the antimicrobial activity with respect to the free form. Likewise, the inhibitory effect is similar to the inhibitory effect of silicon oxide particles functionalized with these natural compounds. Therefore, microcrystalline cellulose can also be used as a solid support for the immobilization of the active compounds of interest.

Other cellulose-rich supports, such as wood, can also be functionalized in order to confer to them the same properties.

### Example 11: Antimicrobial filters

Studies were also performed to develop surfaces with antimicrobial activity after the immobilization of the active compounds. First commercial cellulose paper filters functionalized with thymol were developed. To that end, the paper was impregnated with a solution consisting of the trialkoxysilane derivative and the active compound. After drying the solution on the paper for 2 h, the filters were washed with water and then dried at room temperature.

Once the functionalized filters were obtained, they were used to filter (by gravity) 10 ml of water contaminated with *Listeria innocua* at an inoculum density of 10⁴ CFU/ml. After that, the microbial load of the filtered samples was determined by means of dilution and plate seeding, obtaining a complete microbial load reduction compared to the control samples (water filtered in non-functionalized paper).

The same principle can be applied for developing cotton filters.

### Example 12: Antimicrobial fabrics

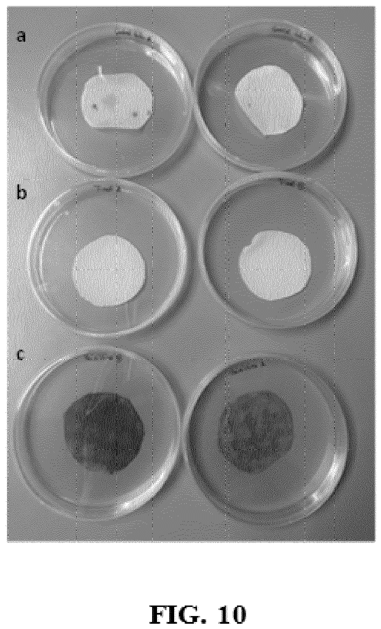

Another type of antimicrobial surface was also developed, these cotton fabrics having antimicrobial activity. The functionalization process is similar to that of the cellulose filters since it consists of impregnation with the activated compounds, washing and drying. The first results of fabrics functionalized with thymol and vanillin showed complete inhibition of *Aspergillus niger* growth compared to the non-functionalized fabric as seen in Figure 10.

### Example 13: Spraying

Aerosols for developing antimicrobial particles or surfaces.

Taking into account the foregoing, it is also possible to develop a spray or aerosol to functionalize supports or surfaces in a quick and simple manner. This aerosol can contain the molecules of interest derivatized with trialkoxysilane and dissolved in a suitable solvent according to the support or surface on which it will be applied.

## Claims

1. An antimicrobial, insecticidal and acaricidal system made up of support surface-functionalized by derivatization with trialkoxysilane groups, with at least one antimicrobial compound of natural origin covalently attached to said support, wherein the antimicrobial compound is selected from the group consisting of carvacrol, cinnamaldehyde, perillaldehyde, eugenol, thymol, vanillin, gallic acid, caprylic acid and ferulic acid.

2. The system according to claim 1, **characterized in that** the antimicrobial, insecticidal and acaricidal compound is selected from the group consisting of carvacrol, eugenol, thymol and vanillin.

3. The system according to any of claims 1 to 2, **characterized in that** the support are particles that are selected from the group consisting of silica-based inorganic materials.

4. The system according to claim 3, **characterized in that** the particles are selected from the group consisting of mesoporous silicon oxide particles with a particle size comprised between 0.01 and 7 µm and a pore size comprised between 1 and 20 nm, and amorphous silicon oxide particles with a particle size comprised between 0.01 and 30 µm.

5. The system according to claim 4, **characterized in that** the particles are mesoporous silicon oxide particles with a particle size comprised between 0.1 and 1.5 µm.

6. The system according to any of claims 4 to 5, **characterized in that** the particles are mesoporous silicon oxide particles with a pore size comprised between 2 and 8 nm.

7. The system according to any of claims 3 to 6, **characterized in that** the particles are selected from MCM-41, SBA-15 and UVM-7.

8. The system according to claim 4, **characterized in that** the particles are amorphous silicon oxide particles with a particle size comprised between 3 and 4 µm.

9. The system according to any of claims 1 to 2, **characterized in that** the support are particles selected from the group consisting of cellulose particles.

10. The system according to claim 9, **characterized in that** the particles are selected from the group consisting of microcrystalline cellulose particles with a particle size comprised between 1 and 10 µm

11. The system according to any of claims 1 to 2, **characterized in that** the functionalized support is selected from the group of cellulose-rich supports.

12. The system according to claim 11, **characterized in that** the cellulose-rich support is selected from the group comprising wood, cotton and paper.

13. The system according to any of the preceding claims, **characterized in that** at least one antimicrobial compound of natural origin is applied to the support by means of spraying.

14. Use of the system according to any of claims 9 to 12 as antimicrobial filters.

15. Use of the system according to any of claims 8 to 12 as antimicrobial fabrics.

## Patentansprüche

1. Antimikrobielles, insektizides und akarizides System, das aus einem durch Derivatisierung mit Trialkoxysilangruppen oberflächenfunktionalisierten Träger besteht, wobei mindestens eine antimikrobielle Verbindung natürlichen Ursprungs kovalent an den Träger gebunden ist, wobei die antimikrobielle Verbindung ausgewählt ist aus der Gruppe, bestehend aus Carvacrol, Zimtaldehyd, Perillaldehyd, Eugenol, Thymol, Vanillin, Gallussäure, Caprylsäure und Ferulasäure.

2. System nach Anspruch 1, **dadurch gekennzeichnet, dass** die antimikrobielle, insektizide und akarizide Verbindung ausgewählt ist aus der Gruppe, bestehend aus Carvacrol, Eugenol, Thymol und Vanillin.

3. System nach einem der Ansprüche 1 bis 2, **dadurch gekennzeichnet, dass** der Träger Partikel sind, die aus der Gruppe, bestehend aus anorganischen Materialien auf Siliciumdioxidbasis, ausgewählt sind.

4. System nach Anspruch 3, **dadurch gekennzeichnet, dass** die Partikel ausgewählt sind aus der Gruppe, bestehend aus mesoporösen Siliciumoxidpartikeln mit einer Partikelgröße zwischen 0,01 und 7 µm und einer Porengröße zwischen 1 und 20 nm und amorphen Siliciumoxidpartikeln mit eine Partikelgröße zwischen 0,01 und 30 µm.

5. System nach Anspruch 4, **dadurch gekennzeichnet, dass** die Partikel mesoporöse Siliciumoxidpartikel mit einer Partikelgröße zwischen 0,1 und 1,5 µm sind.

6. System nach einem der Ansprüche 4 bis 5, **dadurch gekennzeichnet, dass** die Partikel mesoporöse Siliciumoxidpartikel mit einer Porengröße zwischen 2 und 8 nm sind.

7. System nach einem der Ansprüche 3 bis 6, **dadurch gekennzeichnet, dass** die Partikel aus MCM-41, SBA-15 und UVM-7 ausgewählt sind.

8. System nach Anspruch 4, **dadurch gekennzeichnet, dass** die Partikel amorphe Siliciumoxidpartikel mit einer Partikelgröße zwischen 3 und 4 µm sind.

9. System nach einem der Ansprüche 1 bis 2, **dadurch gekennzeichnet, dass** der Träger Partikel sind, die aus der Gruppe, bestehend aus Cellulosepartikel, ausgewählt sind.

10. System nach Anspruch 9, **dadurch gekennzeichnet, dass** die Partikel ausgewählt sind aus der Gruppe, bestehend aus mikrokristallinen Cellulosepartikeln mit einer Partikelgröße zwischen 1 und 10 µm

11. System nach einem der Ansprüche 1 bis 2, **dadurch gekennzeichnet, dass** der funktionalisierte Träger aus der Gruppe von cellulosereichen Trägern ausgewählt ist.

12. System nach Anspruch 11, **dadurch gekennzeichnet, dass** der cellulosereiche Träger aus der Gruppe ausgewählt ist, die Holz, Baumwolle und Papier umfasst.

13. System nach einem der vorhergehenden Ansprüche, **dadurch gekennzeichnet, dass** mindestens eine antimikrobielle Verbindung natürlichen Ursprungs mittels Sprühen auf den Träger aufgebracht wird.

14. Verwendung des Systems nach einem der Ansprüche 9 bis 12 als antimikrobielle Filter.

15. Verwendung des Systems nach einem der Ansprüche 8 bis 12 als antimikrobielle Gewebe.

## Revendications

1. Système antimicrobien, insecticide et acaricide constitué d'un support fonctionnalisé en surface par dérivatisation avec des groupes trialcoxysilane, avec au moins un composé antimicrobien d'origine naturelle lié de manière covalente audit support, dans lequel le composé antimicrobien est choisi dans le groupe constitué par le carvacrol, le cinnamaldéhyde, le périllaldéhyde, l'eugénol, le thymol, la vanilline, l'acide gallique, l'acide caprylique et l'acide férulique.

2. Système selon la revendication 1, **caractérisé en ce que** le composé antimicrobien, insecticide et acaricide est choisi dans le groupe constitué par le carvacrol, l'eugénol, le thymol et la vanilline.

3. Système selon l'une quelconque des revendications 1 à 2, **caractérisé en ce que** le support est constitué de particules choisies dans le groupe constitué par des matériaux inorganiques à base de silice.

4. Système selon la revendication 3, **caractérisé en ce que** les particules sont choisies dans le groupe constitué par des particules d'oxyde de silicium mésoporeux avec une taille de particule comprise entre 0,01 et 7 µm et une taille de pore comprise entre 1 et 20 nm, et des particules d'oxyde de silicium amorphe avec une taille de particule comprise entre 0,01 et 30 µm.

5. Système selon la revendication 4, **caractérisé en ce que** les particules sont des particules d'oxyde de silicium mésoporeux avec une taille de particule comprise entre 0,1 et 1,5 µm.

6. Système selon l'une quelconque des revendications 4 à 5, **caractérisé en ce que** les particules sont des particules d'oxyde de silicium mésoporeux avec une taille de pore comprise entre 2 et 8 nm.

7. Système selon l'une quelconque des revendications 3 à 6, **caractérisé en ce que** les particules sont choisies parmi MCM-41, SBA-15 et UVM-7.

8. Système selon la revendication 4, **caractérisé en ce que** les particules sont des particules d'oxyde de silicium amorphe avec une taille de particule comprise entre 3 et 4 µm.

9. Système selon l'une quelconque des revendications 1 à 2, **caractérisé en ce que** les supports sont des particules choisies dans le groupe constitué par des particules de cellulose.

10. Système selon la revendication 9, **caractérisé en ce que** les particules sont choisies dans le groupe constitué par des particules de cellulose microcristalline avec une taille de particule comprise entre 1 et 10 µm.

11. Système selon l'une quelconque des revendications 1 à 2, **caractérisé en ce que** le support fonctionnalisé est choisi dans le groupe de supports riches en cellulose.

12. Système selon la revendication 11, **caractérisé en ce que** le support riche en cellulose est choisi dans le groupe comprenant le bois, le coton et le papier.

13. Système selon l'une quelconque des revendications précédentes, **caractérisé en ce qu'**au moins un composé antimicrobien d'origine naturelle est appliqué sur le support au moyen d'une pulvérisation.

14. Utilisation du système selon l'une quelconque des revendications 9 à 12 en tant que filtres antimicrobiens.

15. Utilisation du système selon l'une quelconque des revendications 8 à 12 en tant qu'étoffes antimicrobiennes.
